(19) Europäisches Patentamt / European Patent Office / Office européen des brevets

(11) **EP 4 119 052 A1**

(12) **EUROPEAN PATENT APPLICATION**
published in accordance with Art. 153(4) EPC

(43) Date of publication:
**18.01.2023 Bulletin 2023/03**

(21) Application number: **21768099.0**

(22) Date of filing: **03.03.2021**

(51) International Patent Classification (IPC):
**A61B 5/16** (2006.01)      **A61B 5/08** (2006.01)
**A61B 5/113** (2006.01)

(52) Cooperative Patent Classification (CPC):
**A61B 5/08; A61B 5/113; A61B 5/16**

(86) International application number:
**PCT/JP2021/008238**

(87) International publication number:
**WO 2021/182249 (16.09.2021 Gazette 2021/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.03.2020 JP 2020042355**

(71) Applicant: **Panasonic Intellectual Property
Management Co., Ltd.
Osaka-shi, Osaka 540-6207 (JP)**

(72) Inventor: **SUZUKA, Yuko
Osaka-shi, Osaka 540-6207 (JP)**

(74) Representative: **Appelt, Christian W.
Boehmert & Boehmert
Anwaltspartnerschaft mbB
Pettenkoferstrasse 22
80336 München (DE)**

(54) **SIGNAL PROCESSING SYSTEM, SENSOR SYSTEM, BIOLOGICAL MANAGEMENT SYSTEM, ENVIRONMENT CONTROL SYSTEM, SIGNAL PROCESSING METHOD, AND PROGRAM**

(57)    The problem to be overcome by the present disclosure is to provide a signal processing system, a sensor system, a biometric management system, an environmental control system, a signal processing method, and a program, all of which improve the versatility of the system to the point of enabling recognizing the condition of a person's autonomic nervous system. A signal processing system (2) includes an expiratory information acquisition unit (21) and a nerve recognition unit (23). The expiratory information acquisition unit (21) acquires expiratory information as a piece of biometric information about a person's (HI) expiratory volume. The nerve recognition unit (23) recognizes, based on a dispersion in the expiratory information, a condition of the person's (HI) autonomic nervous system.

*FIG. 1*

**Description**

**Technical Field**

The present disclosure relates to a signal processing system, a sensor system, a biometric management system, an environmental control system, a signal processing method, and a program.

**Background Art**

A mental activity determination apparatus is known in the art as an apparatus for determining a human mental activity by using the fact that there is correlation between the result of a frequency analysis on interbeat interval data and the respective degrees of activity of the sympathetic nervous system and the parasympathetic nervous system (see, for example, Patent Literature 1). The mental activity determination apparatus detects the interbeat interval and calculates a variance of the interbeat interval data thus detected and the average value of the interbeat interval or the heart rate. The mental activity determination apparatus determines the subject's mental activity based on the variance of the interbeat interval and either the average interbeat interval or the average heart rate.

The mental activity determination apparatus uses an electrocardiogram detector for measuring the subject' electrocardiogram signal in order to detect the interbeat interval. In this case, the subject wears electrodes for detecting a potential signal. Alternatively, a pulse wave sensor is attached to one of the subject's ears or hands to detect the pulse wave.

However, to detect a person's (subject's) interbeat interval as in the mental activity determination apparatus described above, a high-accuracy heartbeat sensor such as an electrocardiogram detector must be used, thus making the system much less versatile.

**Citation List**

**Patent Literature**

Patent Literature 1: JP H08-280637 A

**Summary of Invention**

It is therefore an object of the present disclosure to provide a signal processing system, a sensor system, a biometric management system, an environmental control system, a signal processing method, and a program, all of which are configured or designed to improve the versatility of the system to the point of enabling recognizing the condition of a person's autonomic nervous system.

A signal processing system according to an aspect of the present disclosure includes: an expiratory information acquisition unit that acquires expiratory information as a piece of biometric information about a person's expiratory volume; and a nerve recognition unit that recognizes, based on a dispersion in the expiratory information, a condition of the person's autonomic nervous system.

A sensor system according to another aspect of the present disclosure includes: the signal processing system described above; and a biometric sensor that detects the expiratory information and outputs the expiratory information to the signal processing system.

A biometric management system according to still another aspect of the present disclosure includes: the sensor system described above; and a biometric condition determination unit that determines, based on a result of recognition made by the nerve recognition unit, the person's mental and/or physical condition(s) as a biometric condition.

An environmental control system according to yet another aspect of the present disclosure includes: the biometric management system described above; and an equipment controller that controls, based on a decision made by the biometric condition determination unit, equipment for changing an environment in a space where the person is present.

A signal processing method according to yet another aspect of the present disclosure includes: an expiratory information acquisition step including acquiring expiratory information as a piece of biometric information about a person's expiratory volume; and a nerve recognition step including recognizing, based on a dispersion in the expiratory information, a condition of the person's autonomic nervous system.

A program according to yet another aspect of the present disclosure is designed to cause a computer system to perform the signal processing method described above.

**Brief Description of Drawings**

FIG. 1 is a block diagram illustrating a sensor system including a signal processing system according to an exemplary embodiment;
FIG. 2 is a block diagram illustrating a first example of the sensor system;
FIG. 3 is a block diagram illustrating a second example of the sensor system;
FIG. 4 is a block diagram illustrating a third example of the sensor system;
FIG. 5 is a graph showing coefficients of determination of a predicted LF/HF ratio and a measured LF/HF ratio in the sensor system;
FIG. 6 is a block diagram illustrating a biometric management system according to the exemplary embodiment;
FIG. 7 is a block diagram illustrating an environmental control system according to the exemplary embodiment;
FIG. 8 is a perspective view illustrating an exemplary arrangement of pieces of equipment in the environmental control system; and
FIG. 9 is a flowchart showing the procedure of a signal processing method according to the exemplary embodiment.

**Description of Embodiments**

The exemplary embodiment to be described below generally relates to a signal processing system, a sensor system, a biometric management system, an environmental control system, a signal processing method, and a program. More particularly, the exemplary embodiment relates to a signal processing system, a sensor system, a biometric management system, an environmental control system, a signal processing method, and a program, all of which are configured or designed to recognize the condition of a person's autonomic nervous system.

Note that the embodiment to be described below is only an exemplary one of various embodiments of the present disclosure and should not be construed as limiting. Rather, the exemplary embodiment may be readily modified in various manners depending on a design choice or any other factor without departing from the scope of the present disclosure.

(Embodiment)

(1) Overview of sensor system

It is well known in the art that it is useful to recognize the condition of a person's autonomic nervous system in order to determine his or her mental and/or physical condition(s) as a biometric condition.

Specifically, an LF/HF ratio is used as an index to the condition of a person's autonomic nervous system. The LF/HF ratio may be obtained based on time series data of RRI (RR interval). As used herein, the "RRI" refers to a time interval between one R wave and the next R wave on a person's electrocardiogram, i.e., the person's interbeat interval. The RRI constantly varies, and the distribution of the varying frequency of the RRI may be calculated as a power spectrum based on the time series data of the RRI In the power spectrum, LF is an integral value of signal strengths in a low-frequency domain (from 0.05 Hz to 0.15 Hz, for example) and HF is an integral value of signal strengths in a high-frequency domain (from 0.15 Hz to 0.40 Hz, for example). The LF/HF ratio when a person's autonomic nervous system is sympathetic dominant is larger than the LF/HF ratio when the person's autonomic nervous system is parasympathetic dominant. For example, the more stressed a person feels, the larger the LF/HF ratio is. The more relaxed a person feels, the smaller the LF/HF ratio is.

To obtain the RRI, however, a person's heartbeat needs to be detected with high resolution and high accuracy. Examples of high-resolution, high-accuracy heartbeat sensors which are currently used in practice include a contact-type sensor for making evaluation either based on an electrocardiogram or by an optical method. However, enabling recognizing the condition of a person's autonomic nervous system without using the heartbeat information as an indispensable one would broaden the range of sensors available for use and improve the system versatility.

With this object in mind, the present inventors carried out an experiment to discover that the LF/HF ratio is closely correlated to a dispersion in a person's end-tidal carbon dioxide tension (ET $CO_2$). The result of the experiment suggested that as the LF/HF ratio increased to make the person more stressed out, the ET $CO_2$ tended to be further dispersed.

As a result of such an experiment, the present inventors discovered that there is close correlation between the dispersion in a person's exhaled breath and the LF/HF ratio. Specifically, the wider the dispersion in a person's exhaled breath is, the higher the LF/HF ratio is. The narrower the dispersion in a person's exhaled breath is, the lower the LF/HF ratio is. In other words, the wider the dispersion in a person's exhaled breath is, the more sympathetic dominant the person's autonomic nervous system is. The narrower the dispersion in a person's exhaled breath is, the more parasympathetic dominant the person's autonomic nervous system is.

Next, a sensor system for recognizing the condition of a person's autonomic nervous system based on this discovery will be described.

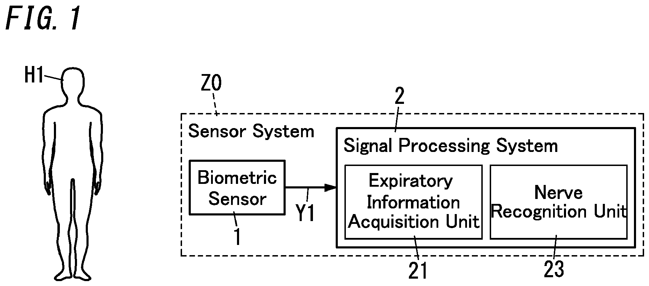

FIG. 1 illustrates a configuration for a sensor system Z0 according to an exemplary embodiment. The sensor system Z0 includes a biometric sensor 1 and a signal processing system 2.

The biometric sensor 1 detects expiratory information as a piece of biometric information about a person's H1 expiratory volume. The biometric sensor 1 outputs an expiratory flow sensor signal Y1 including the expiratory information.

The signal processing system 2 includes an expiratory information acquisition unit 21 and a nerve recognition unit 23.

The expiratory information acquisition unit 21 acquires, from the biometric sensor 1, the expiratory flow sensor signal Y1 including the expiratory information. The communication between the biometric sensor 1 and the expiratory information acquisition unit 21 may be wireless communication or wired communication, whichever is appropriate. The wireless communication is preferably compliant with a protocol such as wireless LAN, Bluetooth®, or ZigBee®. The wired communication is preferably compliant with a wired local area network (LAN) protocol such as Ethernet®. Optionally, the wireless communication and the wired communication may be both compliant with communications protocols dedicated to the signal processing system 2.

The nerve recognition unit 23 obtains the dispersion in the expiratory information as an index to the condition of the person's autonomic nervous system. The nerve recognition unit 23 determines, based on the dispersion in the expiratory information, whether the person's H1 autonomic nervous system is sympathetic dominant or parasympathetic dominant and also determines the degree of the sympathetic or parasympathetic dominance. The dispersion in the expiratory information is preferably at least one of a deviation, a variance, or a standard deviation of the expiratory information.

This signal processing system 2 uses the person's H1 expiratory information, and therefore, does not have to use information about the person's interbeat interval. Thus, the signal processing system 2 may improve the system versatility to the point of enabling recognizing the condition of the person's H1 autonomic nervous system. In addition, the signal processing system 2 uses the dispersion in the expiratory information when recognizing the condition of the person's H1 autonomic nervous system, and therefore, may recognize the condition of the person's H1 autonomic nervous system highly accurately.

The signal processing system 2 includes a computer system. The computer system includes a processor and a memory as principal hardware components thereof. Some or all functions of the signal processing system 2 may be performed by making the processor execute a program stored in the memory. The program may be stored in advance in the memory of the computer system. Alternatively, the program may also be downloaded through a telecommunications line or be distributed after having been recorded in some non-transitory storage medium such as a memory card, an optical disc, or a hard disk drive, any of which is readable for the computer system. The processor of the computer system may be implemented as a single or a plurality of electronic circuits including a semiconductor integrated circuit (IC) or a large-scale integrated circuit (LSI). Those electronic circuits may be either integrated together on a single chip or distributed on multiple chips, whichever is appropriate. Those multiple chips may be aggregated together in a single device or distributed in multiple devices without limitation.

(2) First example of sensor system

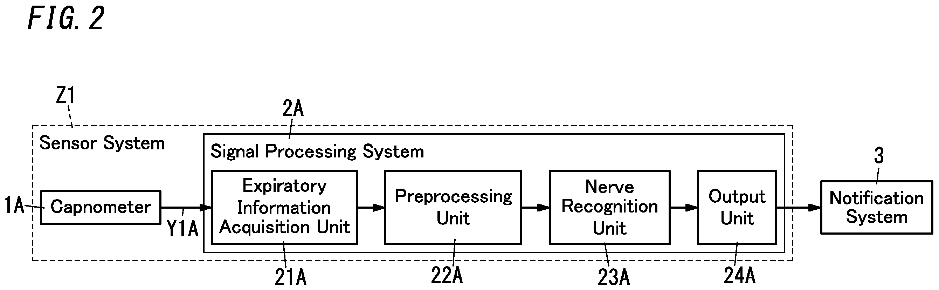

A sensor system Z1 as shown in FIG. 2 will be described as a first example of the sensor system Z0.

As described above, the present inventors discovered that there is close correlation between the dispersion in the carbon dioxide content of a person's H1 exhaled breath and a variation in RRI Thus, this sensor system Z1 includes a capnometer 1A as an exemplary biometric sensor 1 and a signal processing system 2A as an exemplary signal processing system 2.

(2.1) Biometric sensor

As the biometric sensor 1, a $CO_2$ sensor for detecting, as the expiratory information, the carbon dioxide content in a person's H1 exhaled breath is preferably used. The carbon dioxide content in the exhaled breath increases as the person's H1 expiratory volume increases. In particular, the $CO_2$ sensor is preferably the capnometer 1A shown in FIG. 2 for detecting the person's H1 ET $CO_2$. The capnometer 1A detects (measures) the person's H1 ETCO$_2$ based on the exhaled breath supplied via a tube from the person's H1 trachea. The ET $CO_2$ corresponds to the content (concentration) of carbon dioxide included in the person's H1 exhaled breath. The time integral of the carbon dioxide concentration is the carbon dioxide content. The capnometer 1A outputs a result of detection of ET $CO_2$ as an expiratory flow sensor signal Y1A.

(2.2) Signal processing system

As shown in FIG. 2, the signal processing system 2A includes an expiratory information acquisition unit 21A as an exemplary expiratory information acquisition unit 21 and a nerve recognition unit 23A as an exemplary nerve recognition unit 23. The signal processing system 2A preferably further includes a preprocessing unit 22A and an output unit 24A.

The expiratory information acquisition unit 21A acquires, from the capnometer 1A, an expiratory flow sensor signal Y1A, including information about the person's H1 ET $CO_2$, as the expiratory information.

The preprocessing unit 22A has an amplification capability of amplifying the expiratory flow sensor signal Y1A and an AD conversion capability of converting the expiratory flow sensor signal Y1A into a digital signal. The preprocessing unit 22A generates a digital expiratory flow sensor signal including a large number of sample values of ET $CO_2$ generated at a predetermined sampling frequency (ET $CO_2$ measured values). Optionally, the capnometer 1A may perform the function of the preprocessing unit 22A.

The nerve recognition unit 23A receives, from the preprocessing unit 22A, the digital expiratory flow sensor signal and generates, based on the large number of sample values of the ET $CO_2$, time series data of the magnitude of ET $CO_2$. The nerve recognition unit 23A obtains, based on the time series data of the magnitude of ET $CO_2$, the dispersion in the magnitude of the ET $CO_2$ (as an index to the condition of the person's autonomic nervous system). Specifically, the nerve recognition unit 23A obtains, based on the respective sample values of ET $CO_2$ during a first period, an integral of ET $CO_2$ during the first period and uses the integral as the magnitude of ET $CO_2$. Then, the nerve recognition unit 23A obtains, as a dispersion in the magnitude of ET $CO_2$, at least one of a deviation, a variance, or a standard deviation of the magnitude of ET $CO_2$. The nerve recognition unit 23A determines that the more dispersed the magnitude of ET $CO_2$ is, the more sympathetic dominant the person's autonomic nervous system should be. The nerve recognition unit 23A also determines that the less dispersed the magnitude of ET $CO_2$ is, the more parasympathetic dominant the person's autonomic nervous system should be. The duration of the first period is preferably about five minutes, for example, but is not limited to any particular value. Also, the sample period of the time series data of the magnitude of ET $CO_2$ is preferably equal to or shorter than one second.

In addition, it is also preferable that the nerve recognition unit 23A obtain the dispersion in the magnitude of ET $CO_2$ over a second period, which is longer than the first period, and use, as a reference value, the average value of the dispersion in the magnitude of ET $CO_2$ during the second period. In that case, the nerve recognition unit 23A determines that the larger the excess of the dispersion in the magnitude of ET $CO_2$ over the reference value is, the more sympathetic dominant the person's autonomic nervous system should be. The nerve recognition unit 23A also determines that the larger the deficit of the dispersion in the magnitude of ET $CO_2$ under the reference value is, the more parasympathetic dominant the person's autonomic nervous system should be. Note that the duration of the second period is preferably about 24 hours but is not limited to any particular value.

Alternatively, the nerve recognition unit 23A may obtain the dispersion in the magnitude of ET $CO_2$ over a third period, which is longer than the first period, and use, as a differential value, the difference between the maximum and minimum dispersions in the magnitude of ET $CO_2$ during the third period. In that case, the nerve recognition unit 23A may evaluate, based on the magnitude of the differential value, the person's H1 cognitive function. As used herein, evaluation of the person's H1 cognitive function refers to, for example, evaluating the degree of his or her mild cognitive impairment (MCI) or the degree of his or her cognitive impairment. The nerve recognition unit 23A may determine that there could be a decline in his or her cognitive function if the differential value is short of a normal range, for example.

The output unit 24A outputs the result of recognition made by the nerve recognition unit 23A to a notification system 3. The communication between the output unit 24A and the notification system 3 may be wireless communication or wired communication, whichever is appropriate. The wireless communication is preferably compliant with a protocol such as wireless LAN, Bluetooth®, or ZigBee®. The wired communication is preferably compliant with a wired local area network (LAN) protocol such as Ethernet®. Optionally, the wireless communication and the wired communication may be both compliant with communications protocols dedicated to the signal processing system 2A.

The notification system 3 includes at least one of a display device, audio equipment, or any other type of output device. The display device may be, for example, a liquid crystal display or an organic electroluminescent (EL) display and displays the result of recognition made by the nerve recognition unit 23A. The audio equipment includes a loudspeaker and outputs, as a voice message, the result of recognition made by the nerve recognition unit 23A. The notification system 3 is preferably a personal computer, a tablet computer, a smartphone, or a loudspeaker used by an administrator, for example.

This signal processing system 2A uses the capnometer 1A, thereby improving the system versatility to the point of enabling recognizing the condition of the person's autonomic nervous system. In addition, this signal processing system 2A uses a carbon dioxide content (such as ET $CO_2$) that is closely correlated to an LF/HF ratio when recognizing the condition of the person's H1 autonomic nervous system, thus enabling recognizing the condition of a person's H1 autonomic nervous system highly accurately.

(3) Second example of sensor system

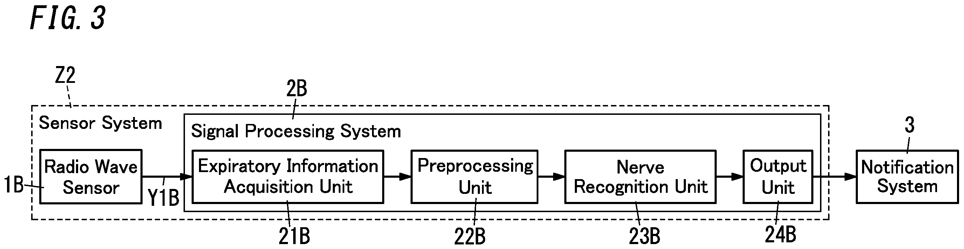

A sensor system Z2 as shown in FIG. 3 will be described as a second example of the sensor system Z0.

As described above, the present inventors discovered that there is close correlation between the dispersion in the carbon dioxide content of a person's H1 exhaled breath and a variation in RRI. Thus, this sensor system Z2 detects the person's H1 body motion which is closely correlated to the carbon dioxide content of the person's H1 exhaled breath. The sensor system Z2 detects, as the person's H1 body motion, the displacement of the person's H1 body. In particular, the sensor system Z2 preferably detects, as the displacement of the person's H1 body, the displacement of at least one of his or her breast, stomach, or back.

Thus, the sensor system Z2 includes a radio wave sensor 1B as an exemplary biometric sensor 1 and a signal processing system 2B as an exemplary signal processing system 2.

(3.1) Biometric sensor

The sensor system Z2 uses the radio wave sensor 1B as the biometric sensor 1.

The radio wave sensor 1B sends out a radio wave as a transmission wave toward a person H1 present within an irradiation area, receives, as a reception wave, the radio wave reflected from the person HI, and outputs an expiratory flow sensor signal Y1B including information about the distance between the radio wave sensor 1B and the person HI. The distance to the person H1 varies according to the body motion of the person H1. Subjecting the expiratory flow sensor signal Y1B including information about the distance, for example, to signal processing enables deriving, as biometric information about the person HI, body motion information such as information about his or her respiration, heartbeat, and pulsation.

The radio wave sensor 1B only needs to be a radio wave sensor with the ability to measure the distance to the person H1 The radio wave sensor 1B may be a frequency-modulated continuous-wave (FMCW) radio wave sensor, a binary frequency shift keying (FSK) radio wave sensor, or any other suitable sensor. Alternatively, if the target of measurement is a single target of measurement, then the radio wave sensor 1B may also be a doppler radio wave sensor for generating I/Q data. The doppler radio wave sensor for generating I/Q data may detect the velocity and direction of displacement of the person's H1 body.

The transmission wave sent out from the radio wave sensor 1B is preferably a microwave. In particular, the frequency of the transmission wave preferably falls within either the 24 GHz band or the 48 GHz band. Nevertheless, the transmission wave does not have to be a microwave but may also be a millimeter wave. Thus, the frequency of the transmission wave is not limited to any particular value.

For example, the sensor system Z2 detects the displacement of the person's H1 breast and/or stomach as the displacement of his or her thoracoabdominal region. Specifically, in the sensor system Z2, the radio wave sensor 1B sends out the transmission wave toward the person's H1 thoracoabdominal part from in front of the person H1. The transmission wave is reflected from the person's H1 thoracoabdominal region. Supposing the person's H1 thoracoabdominal region is his or her front side and the person's H1 back is his or her rear side, the person's H1 thoracoabdominal region moves in the forward/backward direction in synch with the person's H1 respiratory cycle. As the person's H1 intake volume increases, his or her thoracoabdominal region moves forward. As the person's H1 expiratory volume increases, his or her thoracoabdominal region moves backward. Also, as the person's H1 expiratory volume increases, the carbon dioxide content of his or her exhaled breath increases. Therefore, the larger the degree of displacement of the thoracoabdominal region when the person H1 exhales is, the larger the carbon dioxide content of the person's H1 exhaled breath is. In other words, the smaller the degree of displacement of the thoracoabdominal region when the person H1 exhales is, the smaller the carbon dioxide content of the person's H1 exhaled breath is. Thus, information included in the expiratory flow sensor signal Y1B about the distance between the radio wave sensor 1B and the person's H1 thoracoabdominal region is used as information about the displacement of the person's H1 thoracoabdominal region. That is to say, the expiratory flow sensor signal Y1B includes information about the carbon dioxide content that is closely correlated to the LF/HF ratio.

(3.2) Signal processing system

As shown in FIG. 3, the signal processing system 2B includes an expiratory information acquisition unit 21B as an exemplary expiratory information acquisition unit 21 and a nerve recognition unit 23B as an exemplary nerve recognition unit 23. The signal processing system 2B preferably further includes a preprocessing unit 22B and an output unit 24B.

The expiratory information acquisition unit 21B acquires, from the radio wave sensor 1B, an expiratory flow sensor signal Y1B, including information about the distance to the person HI, as the expiratory information.

The preprocessing unit 22B has an amplification capability of amplifying the expiratory flow sensor signal Y1B and an AD conversion capability of converting the expiratory flow sensor signal Y1B into a digital signal. The preprocessing unit 22B generates a digital expiratory flow sensor signal including a large number of sample values of the distance generated at a predetermined sampling frequency (distance measured values). Optionally, the radio wave sensor 1B may perform the function of the preprocessing unit 22B.

The nerve recognition unit 23B receives the digital expiratory flow sensor signal from the preprocessing unit 22B and generates, based on the large number of sample values of the distance, time series data about the displacement of the thoracoabdominal region when the person H1 exhales. Specifically, the nerve recognition unit 23B subtracts a local maximum value of the distance (i.e., the distance when the person H1 exhales) from a local minimum value of the distance (i.e., the distance when the person H1 inhales) and defines the difference thus calculated to be the displacement of the person's H1 thoracoabdominal region. Then, the nerve recognition unit 23B obtains, as a dispersion in the displacement of the thoracoabdominal region (which is an index to the condition of the person's autonomic nervous system), at least one of a deviation, a variance, or a standard deviation of the displacement of the thoracoabdominal region in the eleventh period. In that case, the nerve recognition unit 23B determines that the more dispersed the displacement of the thoracoabdominal region is, the more sympathetic dominant the person's autonomic nervous system should be. The nerve recognition unit 23B also determines that the less dispersed the displacement of the thoracoabdominal region is, the more parasympathetic dominant the person's autonomic nervous system should be. Note that the duration of the eleventh period is preferably about five minutes but is not limited to any particular value. Also, the sample period of the time series data about the displacement of the thoracoabdominal region is preferably equal to or shorter than one second.

In addition, it is also preferable that the nerve recognition unit 23B obtain the dispersion in the displacement of the thoracoabdominal region at regular intervals over the twelfth period, which is longer than the eleventh period, and use, as a reference value, the average value of the dispersion in the displacement of the thoracoabdominal region during the twelfth period. In that case, the nerve recognition unit 23B determines that the larger the excess of the dispersion in the displacement of the thoracoabdominal region over the reference value is, the more sympathetic dominant the person's autonomic nervous system should be. The nerve recognition unit 23B also determines that the larger the deficit of the dispersion in the displacement of the thoracoabdominal region under the reference value is, the more parasympathetic dominant the person's autonomic nervous system should be. Note that the duration of the twelfth period is preferably about 24 hours but is not limited to any particular value.

Alternatively, the nerve recognition unit 23B may obtain the dispersion in the displacement of the thoracoabdominal region over a thirteenth period, which is longer than the eleventh period, and use, as a differential value, the difference between the maximum and minimum dispersions in the displacement of the thoracoabdominal region during the thirteenth period. In that case, the nerve recognition unit 23B may evaluate, based on the magnitude of the differential value, the person's H1 cognitive function.

The output unit 24B outputs the result of recognition made by the nerve recognition unit 23B to a notification system 3. The communication between the output unit 24B and the notification system 3 may be wireless communication or wired communication, whichever is appropriate. The wireless communication is preferably compliant with a protocol such as wireless LAN, Bluetooth®, or ZigBee®. The wired communication is preferably compliant with a wired local area network (LAN) protocol such as Ethernet®. Optionally, the wireless communication and the wired communication may be both compliant with communications protocols dedicated to the signal processing system 2B.

The notification system 3 includes at least one of a display device, audio equipment, or any other type of output device. The notification system 3 makes notification of the result of recognition made by the nerve recognition unit 23A.

This signal processing system 2B uses the radio wave sensor 1B, thereby improving the system versatility to the point of enabling recognizing the condition of the person's autonomic nervous system. In addition, this signal processing system 2B uses the displacement of the thoracoabdominal region that is closely correlated to an LF/HF ratio when recognizing the condition of the person's H1 autonomic nervous system, thus enabling recognizing the condition of the person's H1 autonomic nervous system highly accurately.

Furthermore, the nerve recognition unit 23B preferably further detects, based on the digital expiratory flow sensor signal received from the preprocessing unit 22B, the person's H1 heartbeat. The expiratory flow sensor signal includes not only information about the displacement of the thoracoabdominal region due to the person's H1 respiration but also information about the displacement due to the person's H1 heartbeat. The radio wave sensor 1B serves as not only a biometric sensor for detecting the person's H1 expiratory information but also a heartbeat sensor for detecting the person's H1 heartbeat. The nerve recognition unit 23B generates, based on the expiratory flow sensor signal, not only the time series data about the displacement of the person's H1 thoracoabdominal region but also time series data about the person's H1 heartbeat as well. Then, the nerve recognition unit 23B determines, based on the dispersion in the displacement of the thoracoabdominal region and the dispersion in the heart rate per predetermined time, whether the person's H1 autonomic nervous system is sympathetic dominant or parasympathetic dominant and also determines the degree of the sympathetic or parasympathetic dominance. The heart rate per predetermined time may be, for example, a heart rate per minute. However, this is only an example and the predetermined time does not have to be one minute.

For example, a prediction formula for predicting the LF/HF ratio based on the dispersion in the displacement of the thoracoabdominal region and the dispersion in the heart rate may be obtained by performing a multiple regression analysis using the dispersion in the displacement of the thoracoabdominal region and the dispersion in the heart rate as respective explanatory variables and using the LF/HF ratio as a reference variable. The nerve recognition unit 23B obtains a predicted LF/HF ratio by substituting the dispersion in the displacement of the thoracoabdominal region and the dispersion in the heart rate into this prediction formula. In that case, the nerve recognition unit 23B determines, based on the predicted LF/HF ratio, whether the person's H1 autonomic nervous system is sympathetic dominant or parasympathetic dominant and also determines the degree of the sympathetic or parasympathetic dominance.

The condition of the person's H1 autonomic nervous system is recognized by using both the dispersion in the displacement of the thoracoabdominal region and the dispersion in the heart rate as described above, thus enabling performing recognition processing with even more accuracy.

Note that in the sensor system Z2, if the displacement of the person's H1 back needs to be detected as the displacement of the person's H1 body, then the radio wave sensor 1B sends out a transmission wave from behind the person's H1 back toward his or her back.

(4) Third example of sensor system

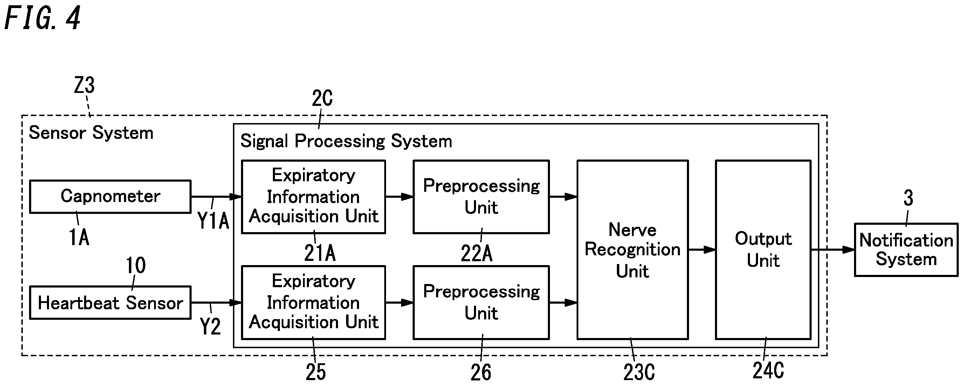

A sensor system Z3 as shown in FIG. 4 will be described as a third example of the sensor system Z0.

The sensor system Z3 includes a capnometer 1A, a heartbeat sensor 10, and a signal processing system 2C as an exemplary signal processing system 2. The signal processing system 2C includes not only the expiratory information acquisition unit 21A and preprocessing unit 22A of the sensor system Z1 but also a nerve recognition unit 23C, an output unit 24C, a heartbeat information acquisition unit 25, and another preprocessing unit 26 as well.

(4.1) Heartbeat sensor

The heartbeat sensor 10 may be, for example, a contact-type sensor for making evaluation either based on an electrocardiogram or by an optical method and may be attached to the person's H1 breast or wrist, for example. The heartbeat sensor 10 outputs, as a result of detection of the person's H1 heartbeat, a heartbeat sensor signal Y2 including information about the heartbeat waveform.

(4.2) Signal processing system

The heartbeat information acquisition unit 25 acquires a heartbeat sensor signal Y2 from the heartbeat sensor 10. The communication between the heartbeat sensor 10 and the heartbeat information acquisition unit 25 may be wireless communication or wired communication, whichever is appropriate. The wireless communication is preferably compliant with a protocol such as wireless LAN, Bluetooth®, or ZigBee®. The wired communication is preferably compliant with a wired local area network (LAN) protocol such as Ethernet®. Optionally, the wireless communication and the wired communication may be both compliant with communications protocols dedicated to the sensor system Z3.

The preprocessing unit 26 has an amplification capability of amplifying the heartbeat sensor signal Y2 and an AD conversion capability of converting the heartbeat sensor signal Y2 into a digital signal. The preprocessing unit 26 generates a digital heartbeat sensor signal including a large number of sample values of the heartbeat waveform (representing the strength of the heartbeat waveform) at a predetermined sampling frequency. Optionally, the heartbeat sensor 10 may perform the function of the preprocessing unit 26.

The nerve recognition unit 23C receives the digital expiratory flow sensor signal from the preprocessing unit 22A and also receives the digital heartbeat sensor signal from the preprocessing unit 26. The nerve recognition unit 23C generates time series data about the person's H1 magnitude of ET $CO_2$ based on the expiratory flow sensor signal and also generates time series data about the person's H1 heartbeat based on the heartbeat sensor signal. Then, the nerve recognition unit 23C determines, based on the dispersion in the magnitude of the ET $CO_2$ and the dispersion in the heart rate per predetermined time (which are indices to the condition of the person's H1 autonomic nervous system), whether the person's H1 autonomic nervous system is sympathetic dominant or parasympathetic dominant and further determines the degree of the sympathetic or parasympathetic dominance.

For example, a prediction formula for predicting the LF/HF ratio based on the dispersion in the magnitude of ET $CO_2$ and the dispersion in the heart rate may be obtained by performing a multiple regression analysis using the dispersion in the magnitude of the ET $CO_2$ and the dispersion in the heart rate as respective explanatory variables and using the LF/HF ratio as a reference variable. The prediction formula may be expressed, for example, as the following Equation (1):

$$LF/HF = 2.1355 \cdot \sigma1 + 232.6184 \cdot \sigma2 - 11.0781 \qquad (1)$$

where $\sigma 1$ is a standard deviation representing the dispersion in the magnitude of ET $CO_2$ and $\sigma 2$ is a standard deviation representing the dispersion in the heart rate. The nerve recognition unit 23C obtains a predicted LF/HF ratio by the prediction formula expressed by this Equation (1). In that case, the nerve recognition unit 23C determines, based on the predicted LF/HF ratio, whether the person's H1 autonomic nervous system is sympathetic dominant or parasympathetic dominant and also determines the degree of the sympathetic or parasympathetic dominance.

The output unit 24C outputs the result of recognition made by the nerve recognition unit 23C to the notification system 3. The communication between the output unit 24C and the notification system 3 may be wireless communication or wired communication, whichever is appropriate. The wireless communication is preferably compliant with a protocol such as wireless LAN, Bluetooth®, or ZigBee®. The wired communication is preferably compliant with a wired local area network (LAN) protocol such as Ethernet®. Optionally, the wireless communication and the wired communication may be both compliant with communications protocols dedicated to the signal processing system 2C.

This sensor system Z3 recognizes the condition of the person's H1 autonomic nervous system by using both the dispersion in the displacement of the thoracoabdominal region and the dispersion in the heart rate as described above, thus enabling performing recognition processing with even more accuracy.

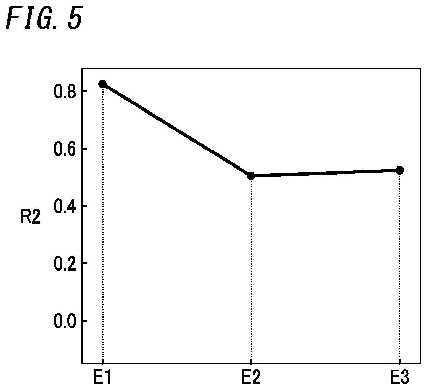

The present inventors sequentially changed the environment surrounding the person H1 from a first environment E1 to a second environment E2 and then to a third environment E3 to obtain the person's H1 predicted LF/HF ratios under the respective environments by Equation (1). Coefficients of determination R2 of predicted LF/HF ratios under the respective environments and measured LF/HF ratios under the respective environments are shown in FIG. 5. In this example, the first environment E1 may be a state where the person H1 is closing his or her eyes in the dark. The second environment E2 may be a state where the person H1 is irradiated with white light with pop music played as background music. The third environment E3 may be a state where classical music is played as background music in the dark. As shown in FIG. 5, the coefficients of determination R2 under the respective environments fall within the range from 0.5 to 0.9. Thus, it can be seen that the predicted LF/HF ratio obtained by Equation (1) is closely correlated to the measured LF/HF ratio (i.e., the accuracy of Equation (1) is relatively high).

Optionally, the sensor system Z2 shown in FIG. 3 may also include the heartbeat sensor 10 separately from the radio wave sensor 1B to recognize the condition of the person's H1 autonomic nervous system by using both the dispersion in the displacement of the thoracoabdominal region and the dispersion in the heart rate.

(5) Biometric management system

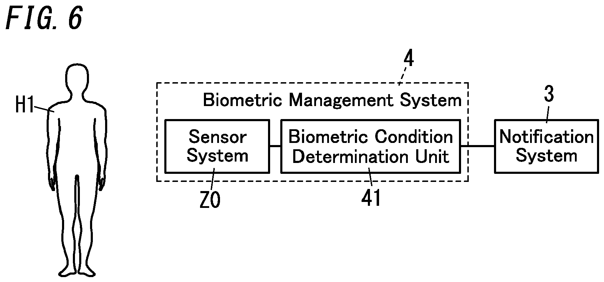

FIG. 6 illustrates a configuration for a biometric management system 4. The biometric management system 4 includes the sensor system Z0 (including any one of the sensor systems Z1-Z3) and a biometric condition determination unit 41.

The biometric condition determination unit 41 receives a result of recognition made by the nerve recognition unit 23 (including any one of the nerve recognition units 23A-23C) of the sensor system Z0. The biometric condition determination unit 41 determines, based on the result of recognition made by the nerve recognition unit 23, the person's H1 mental and/or physical condition(s) as a biometric condition.

Note that the biometric condition determination unit 41 may be implemented as either the same computer system as the sensor system Z0 or a different computer system from the sensor system Z0, whichever is appropriate.

The result of recognition made by the nerve recognition unit 23 includes a decision about the degree of either the sympathetic dominance or the parasympathetic dominance in the person's H1 autonomic nervous system. This allows the biometric condition determination unit 41 to determine, based on the result of recognition made by the nerve recognition unit 23, the person's H1 stress level as a biometric condition. The biometric condition determination unit 41 determines that the larger the degree of the sympathetic dominance is, the more stressed out the person's H1 should be and also determines that the larger the degree of the parasympathetic dominance is, the less stressed out the person's H1 should be. Optionally, the biometric condition determination unit 41 may determine not only the person's H1 stress level but also the degree of the person's H1 cognitive function.

The biometric condition determination unit 41 transmits the decision thus made to the notification system 3. In response, the notification system 3 notifies an administrator, for example, of the decision made by the biometric condition determination unit 41. The notification system 3 is preferably a personal computer, tablet computer, or smartphone used by the administrator, for example.

Optionally, the biometric management system 4 may be installed in a moving vehicle steered by the person HI. Examples of the moving vehicle include automobiles, aircrafts, and watercrafts. The moving vehicle is caused to move by being steered by the person HI. In this case, the biometric condition determination unit 41 determines, based on the result of recognition made by the nerve recognition unit 23, the person's H1 arousal level, thereby making a decision about the person's H1 napping, degree of attention, and/or degree of fatigue as a biometric condition. In that case, the notification system 3 may be a display device and audio equipment in the moving vehicle and is designed to increase the person's H1 arousal level with an image displayed and a sound emitted.

(6) Environmental control system

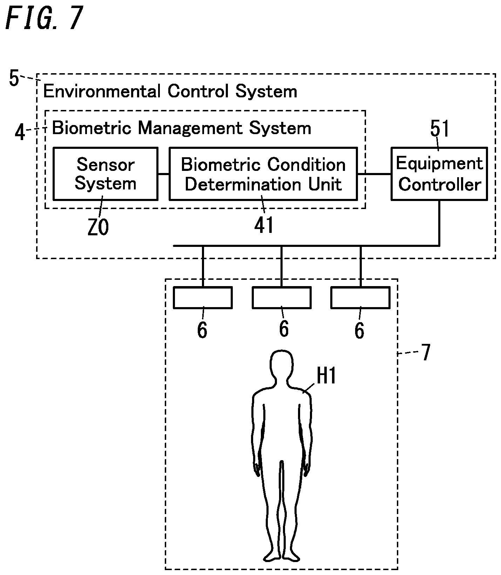

FIG. 7 illustrates a configuration for an environmental control system 5. The environmental control system 5 includes the biometric management system 4 and an equipment controller 51.

The equipment controller 51 receives the decision made by the biometric condition determination unit 41 of the biometric management system 4. The equipment controller 51 controls, based on the decision made by the biometric condition determination unit 41, equipment 6 that changes the environment in a space 7 where the person H1 is present. Note that the equipment controller 51 may be implemented as either the same computer system as the biometric management system 4 or a different computer system from the biometric management system 4, whichever is appropriate.

The decision made by the biometric condition determination unit 41 includes at least one decision made about the person's H1 stress level, the degree of the person's H1 cognitive function, and/or the person's H1 arousal level, for example. Thus, the equipment controller 51 controls, based on the decision made by the biometric condition determination unit 41, the equipment 6 that changes the environment in the space 7. The equipment 6 includes at least one device selected from, for example, the group consisting of an air conditioner, a circulator, a lighting fixture, audio equipment, an aroma diffuser, a display device, a ventilator, and an outside light adjuster (i.e., sunshade). By operating at least one of these pieces of equipment 6, at least one selected from, for example, the group consisting of the temperature, humidity, airflow velocity, illuminance, color of the illuminating light, background music (BGM), aroma, moving picture, $CO_2$ concentration, and incoming light in the space 7 may be controlled.

Thus, the equipment controller 51 may contribute to, for example, relieving the stress the person H1 feels, improving the person's H1 cognitive function, or increasing the person's H1 arousal level by controlling the equipment 6 based on the decision made by the biometric condition determination unit 41.

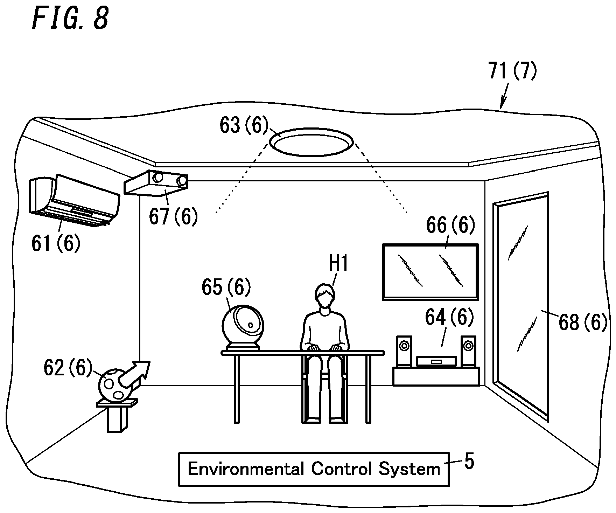

FIG. 8 illustrates, as an exemplary space 7, of which the environment is controlled by the environmental control system 5, a room 71 in which the person H1 is present.

In the room 71, arranged as pieces of equipment 6 are an air conditioner 61, a circulator 62, a lighting fixture 63, audio equipment 64, an aroma diffuser 65, a display device 66, a ventilator 67, and an incoming light adjuster 68. The air conditioner 61 is used to control the temperature and humidity in the room 71. The circulator 62 is used to control the airflow in the room 71. The lighting fixture 63 is used to control the illuminance and light color in the room 71. The audio equipment 64 is used to play background music (BGM) or emit environmental sounds (such as a rain sound, a wind sound, a wave sound, bird's chirrup, and insect's chirping), for example, in the room 71. The aroma diffuser 65 is used to control the aroma in the room 71. The display device 66 is used to display, for example, environmental video (e.g., video of natural scenery such as mountains, oceans, or rivers). The ventilator 67 is used to control the $CO_2$ concentration in the room 71 by ventilating the room 71. The incoming light adjuster 68 may be an electric blind or an electric curtain, for example, and is used to adjust the amount of outside light entering the room 71. The environmental control system 5 controls, based on the decision made by the biometric management system 4, these pieces of equipment 6, thus controlling the stimulus to the person's H1 visual, aural, olfactory, and tactual senses, for example, and also adjusting the temperature and humidity the person H1 feels in the room 71 and thereby contributing to, for example, relieving the stress the person H1 feels, improving his or her cognitive function, and increasing his or her arousal level.

In particular, the environmental control system 5 may effectively control the person's H1 autonomic nervous system by adjusting the temperature and/or humidity in the room 71 with the air conditioner 61 controlled.

(7) Signal processing method

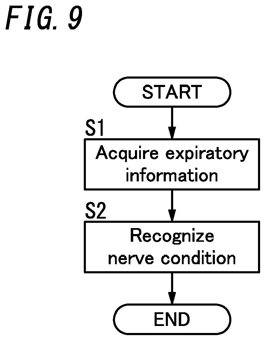

The signal processing method according to this embodiment may be summarized as shown in the flowchart of FIG. 9.

The signal processing method shown in FIG. 9 includes an expiratory information acquisition step S1 and a nerve recognition step S2.

The expiratory information acquisition step S1 includes making the expiratory information acquisition unit 21 acquire expiratory information as a piece of biometric information about a person's H1 expiratory volume.

The nerve recognition step S2 includes making the nerve recognition unit 23 recognize, based on a dispersion in the expiratory information, a condition of the person's H1 autonomic nervous system.

A program stored in a memory of the computer system is preferably designed to cause a processor to perform the signal processing method described above.

The signal processing method and program may also improve the system versatility to the point of enabling recognizing the condition of the person's autonomic nervous system. In addition, the signal processing method and program use the dispersion in the expiratory information when recognizing the condition of the person's H1 autonomic nervous system, and therefore, may recognize the condition of the person's H1 autonomic nervous system highly accurately.

(8) Recapitulation

As can be seen from the foregoing description, a signal processing system (2, 2A, 2B, 2C) according to a first aspect of the exemplary embodiment includes an expiratory information acquisition unit (21, 21A, 21B) and a nerve recognition unit (23, 23A, 23B, 23C). The expiratory information acquisition unit (21, 21A, 21B) acquires expiratory information as a piece of biometric information about a person's (HI) expiratory volume. The nerve recognition unit (23, 23A, 23B, 23C) recognizes, based on a dispersion in the expiratory information, a condition of the person's (HI) autonomic nervous system.

This signal processing system (2, 2A, 2B, 2C) may improve the system versatility to the point of enabling recognizing the condition of a person's (HI) autonomic nervous system. In addition, the signal processing system (2, 2A, 2B, 2C) uses a dispersion in expiratory information when recognizing the condition of the person's (HI) autonomic nervous system, thus enabling recognizing the condition of the person's (HI) autonomic nervous system highly accurately.

In a signal processing system (2, 2A, 2B, 2C) according to a second aspect of the exemplary embodiment, which may be implemented in conjunction with the first aspect, the nerve recognition unit (23, 23A, 23B, 23C) uses, as the dispersion, at least one of a deviation, a variance, or a standard deviation.

This signal processing system (2, 2A, 2B, 2C) may recognize the condition of the person's (HI) autonomic nervous system highly accurately.

A sensor system (Z0, Z1, Z2, Z3) according to a third aspect of the exemplary embodiment includes: the signal processing system (2, 2A, 2B, 2C) according to the first or second aspect; and a biometric sensor (1, 1A, 1B) that detects the expiratory information and outputs the expiratory information to the signal processing system (2, 2A, 2B, 2C).

This sensor system (Z0, Z1, Z2, Z3) may improve the system versatility to the point of enabling recognizing the condition of a person's (HI) autonomic nervous system.

In a sensor system (Z1) according to a fourth aspect of the exemplary embodiment, which may be implemented in conjunction with the third aspect, the biometric sensor (1A) preferably detects, as the expiratory information, a carbon dioxide content of the person's (HI) exhaled breath.

This sensor system (Z1) uses a carbon dioxide content that is closely correlated to an LF/HF ratio, thus enabling recognizing the condition of a person's (HI) autonomic nervous system highly accurately.

In a sensor system (Z1) according to a fifth aspect of the exemplary embodiment, which may be implemented in conjunction with the fourth aspect, the biometric sensor (1A) preferably detects, as the carbon dioxide content, an end-tidal carbon dioxide tension.

This sensor system (Z1) uses an end-tidal carbon dioxide tension that is closely correlated to an LF/HF ratio, thus enabling recognizing the condition of a person's (HI) autonomic nervous system highly accurately.

In a sensor system (Z2) according to a sixth aspect of the exemplary embodiment, which may be implemented in conjunction with the third aspect, the biometric sensor (1B) preferably detects, as the expiratory information, the person's (HI) body motion.

This sensor system (Z2) uses body motion that is closely correlated to an LF/HF ratio, thus enabling recognizing the condition of a person's (HI) autonomic nervous system highly accurately.

In a sensor system (Z2) according to a seventh aspect of the exemplary embodiment, which may be implemented in conjunction with the sixth aspect, the biometric sensor (1B) preferably detects, as the expiratory information, a displacement of the person's (H1) body.

This sensor system (Z2) uses the displacement of the person's (H1) body that is closely correlated to an LF/HF ratio, thus enabling recognizing the condition of a person's (H1) autonomic nervous system highly accurately.

In a sensor system (Z2) according to an eighth aspect of the exemplary embodiment, which may be implemented in conjunction with the sixth or seventh aspect, the biometric sensor (1B) is preferably a radio wave sensor that receives a radio wave reflected from the person (H1).

This sensor system (Z2) may detect the person's (H1) body motion easily.

A sensor system (Z2, Z3) according to a ninth aspect of the exemplary embodiment, which may be implemented in conjunction with any one of the third to eighth aspects, further includes a heartbeat sensor (1B, 10) that detects the person's (H1) heartbeat. The nerve recognition unit (23C) recognizes, based on a dispersion in the expiratory information and the heart rate during a predetermined period, the condition of the person's (H1) autonomic nervous system.

This sensor system (Z2, Z3) may have the recognition processing done with even more accuracy.

A biometric management system (4) according to a tenth aspect of the exemplary embodiment includes: the sensor system (Z0, Z1, Z2, Z3) according to any one of the third to ninth aspects; and a biometric condition determination unit (41) that determines, based on a result of recognition made by the nerve recognition unit (23, 23A, 23B, 23C), the person's (H1) mental and/or physical condition(s) as a biometric condition.

This biometric management system (4) may improve the system versatility to the point of enabling recognizing not only the condition of a person's (H1) autonomic nervous system but also the person's (H1) biometric condition as well.

In a biometric management system (4) according to an eleventh aspect of the exemplary embodiment, which may be implemented in conjunction with the tenth aspect, the biometric condition determination unit (41) preferably determines, as the biometric condition, the person's (HI) stress level.

This biometric management system (4) may determine the person's (H1) stress level easily.

In a biometric management system (4) according to a twelfth aspect of the exemplary embodiment, which may be implemented in conjunction with the tenth aspect, the biometric condition determination unit (41) preferably determines the biometric condition of the person (HI) who is steering a moving vehicle.

This biometric management system (4) may easily determine the arousal level of the person (HI) who is steering a moving vehicle.

An environmental control system (5) according to a thirteenth aspect of the exemplary embodiment includes: the biometric management system (4) according to any one of the tenth to twelfth aspects; and an equipment controller (51) that controls, based on a decision made by the biometric condition determination unit (41), equipment (6) for changing an environment in a space (7) where the person (HI) is present.

This environmental control system (5) may improve the system versatility to the point of enabling recognizing not only the condition of a person's (HI) autonomic nervous system but also the person's (HI) biometric condition as well.

In an environmental control system (5) according to a fourteenth aspect of the exemplary embodiment, which may be implemented in conjunction with the thirteenth aspect, the equipment controller (51) preferably changes at least one of a temperature or a humidity in the space (7) by controlling at least an air conditioner (61) as the equipment (6).

This environmental control system (5) may effectively control the person's (HI) autonomic nervous system.

A signal processing method according to a fifteenth aspect of the exemplary embodiment includes: an expiratory information acquisition step (S1) and a nerve recognition step (S2). The expiratory information acquisition step (S1) includes acquiring expiratory information as a piece of biometric information about a person's (H1) expiratory volume. The nerve recognition step (S2) includes recognizing, based on a dispersion in the expiratory information, a condition of the person's (H1) autonomic nervous system.

This signal processing method may improve the system versatility to the point of enabling recognizing the condition of a person's (H1) autonomic nervous system.

A program according to a sixteenth aspect of the exemplary embodiment is designed to cause a computer system to perform the signal processing method according to the fifteenth aspect.

This program may improve the system versatility to the point of enabling recognizing the condition of a person's (HI) autonomic nervous system.

A signal processing method according to a seventeenth aspect of the exemplary embodiment includes: an expiratory information acquisition step and an index derivation step. The expiratory information acquisition step includes acquiring expiratory information as a piece of biometric information about a person's (HI) expiratory volume. The index derivation step includes deriving a dispersion in the expiratory information as an index to a condition of the person's (HI) autonomic nervous system.

This signal processing method may improve the system versatility to the point of enabling deriving an index to the condition of a person's (HI) autonomic nervous system.


**Reference Signs List**

  1 Biometric Sensor
  1A Capnometer (Biometric Sensor)
  1B Radio Wave Sensor (Biometric Sensor) (Heartbeat Sensor)
  2, 2A, 2B, 2C Signal Processing System
  21, 21A, 21B Expiratory Information Acquisition Unit
  23, 23A, 23B, 23C Nerve Recognition Unit
  4 Biometric Management System
  41 Biometric Condition Determination Unit
  5 Environmental Control System
  51 Equipment Controller
  6 Equipment
  61 Air Conditioner
  7 Space
  10 Heartbeat Sensor
  H1 Person
  Z0, Z1, Z2, Z3 Sensor System
  S1 Expiratory Information Acquisition Step

S2 Nerve Recognition Step

**Claims**

1. A signal processing system comprising:

   an expiratory information acquisition unit configured to acquire expiratory information as a piece of biometric information about a person's expiratory volume; and
   a nerve recognition unit configured to recognize, based on a dispersion in the expiratory information, a condition of the person's autonomic nervous system.

2. The signal processing system of claim 1, wherein
   the nerve recognition unit is configured to use, as the dispersion, at least one of a deviation, a variance, or a standard deviation.

3. A sensor system comprising:

   the signal processing system of claim 1 or 2; and
   a biometric sensor configured to detect the expiratory information and output the expiratory information to the signal processing system.

4. The sensor system of claim 3, wherein
   the biometric sensor is configured to detect, as the expiratory information, a carbon dioxide content of the person's exhaled breath.

5. The sensor system of claim 4, wherein
   the biometric sensor is configured to detect, as the carbon dioxide content, an end-tidal carbon dioxide tension.

6. The sensor system of claim 3, wherein
   the biometric sensor is configured to detect, as the expiratory information, the person's body motion.

7. The sensor system of claim 6, wherein
   the biometric sensor is configured to detect, as the expiratory information, a displacement of the person's body.

8. The sensor system of claim 6 or 7, wherein
   the biometric sensor is a radio wave sensor configured to receive a radio wave reflected from the person.

9. The sensor system of any one of claims 3 to 8, further comprising a heartbeat sensor configured to detect the person's heartbeat, wherein
   the nerve recognition unit is configured to recognize, based on a dispersion in the expiratory information and the heart rate during a predetermined period, the condition of the person's autonomic nervous system.

10. A biometric management system comprising:

    the sensor system of any one of claims 3 to 9; and
    a biometric condition determination unit configured to determine, based on a result of recognition made by the nerve recognition unit, the person's mental and/or physical condition(s) as a biometric condition.

11. The biometric management system of claim 10, wherein
    the biometric condition determination unit is configured to determine, as the biometric condition, the person's stress level.

12. The biometric management system of claim 10, wherein
    the biometric condition determination unit is configured to determine the biometric condition of the person who is steering a moving vehicle.

13. An environmental control system comprising:

the biometric management system of any one of claims 10 to 12; and
an equipment controller configured to control, based on a decision made by the biometric condition determination unit, equipment configured to change an environment in a space where the person is present.

**14.** The environmental control system of claim 13, wherein
the equipment controller is configured to change at least one of a temperature or a humidity in the space by controlling at least an air conditioner as the equipment.

**15.** A signal processing method comprising:

an expiratory information acquisition step including acquiring expiratory information as a piece of biometric information about a person's expiratory volume; and
a nerve recognition step including recognizing, based on a dispersion in the expiratory information, a condition of the person's autonomic nervous system.

**16.** A program designed to cause a computer system to perform the signal processing method of claim 15.

## FIG. 1

H1

Z0

Sensor System

2

Signal Processing System

Biometric Sensor

1

Y1

Expiratory Information Acquisition Unit

21

Nerve Recognition Unit

23

# FIG. 2

*FIG. 3*

Z2

2B

Sensor System

Signal Processing System

1B — Radio Wave Sensor — Y1B → Expiratory Information Acquisition Unit → Preprocessing Unit → Nerve Recognition Unit → Output Unit → Notification System

21B  22B  23B  24B

EP 4 119 052 A1

FIG. 4

FIG. 5

*FIG. 6*

## FIG. 7

Environmental Control System

Biometric Management System

Sensor System

Biometric Condition Determination Unit

Equipment Controller

Z0

41

51

5

4

6 6 6

7

H1

FIG. 8

71 (7)

63 (6)

67 (6)

61 (6)

66 (6)

H1

65 (6)

64 (6)

68 (6)

62 (6)

Environmental Control System 5

*FIG. 9*

```
        ┌─────────────────┐
        │      START      │
        └────────┬────────┘
    S1           │
        ┌────────▼────────┐
        │ Acquire expiratory│
        │   information   │
        └────────┬────────┘
    S2           │
        ┌────────▼────────┐
        │    Recognize    │
        │  nerve condition│
        └────────┬────────┘
                 │
        ┌────────▼────────┐
        │       END       │
        └─────────────────┘
```

**EP 4 119 052 A1**

**INTERNATIONAL SEARCH REPORT**

<table>
<tr><td colspan="2">International application No.</td></tr>
<tr><td colspan="2">PCT/JP2021/008238</td></tr>
</table>

**A. CLASSIFICATION OF SUBJECT MATTER**
Int.Cl. A61B5/16(2006.01)i, A61B5/08(2006.01)i, A61B5/113(2006.01)i
FI: A61B5/16110, A61B5/08, A61B5/113

According to International Patent Classification (IPC) or to both national classification and IPC

**B. FIELDS SEARCHED**

Minimum documentation searched (classification system followed by classification symbols)
Int.Cl. A61B5/16-5/18, A61B5/08-5/097, A61B5/11-5/113

Documentation searched other than minimum documentation to the extent that such documents are included in the fields searched
Published examined utility model applications of Japan     1922-1996
Published unexamined utility model applications of Japan   1971-2021
Registered utility model specifications of Japan           1996-2021
Published registered utility model applications of Japan   1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2011-15887 A (MITSUBISHI ELECTRIC CORPORATION) 27 January 2011 (2011-01-27), paragraphs [0029], [0031], [0032], [0052], [0088], [0105], [0136], [0137] | 1-3, 6-10, 13-16<br>4-5, 11-12 |
| X<br>Y | JP 2011-94881 A (MITSUBISHI ELECTRIC CORPORATION) 12 May 2011 (2011-05-12), paragraphs [0017], [0108], [0110], [0111], [0129], [0184] | 1-3, 6-10, 13-16<br>4-5, 11-12 |
| Y | JP 2005-52413 A (SOPHIA CO., LTD.) 03 March 2005 (2005-03-03), paragraph [0090] | 4-5 |

☒ Further documents are listed in the continuation of Box C.     ☒ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>11 May 2021 | Date of mailing of the international search report<br>18 May 2021 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

24

**INTERNATIONAL SEARCH REPORT**

| International application No. |
| --- |
| PCT/JP2021/008238 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | 久保井 諒，広帯域レーダを用いた呼吸モニタリングにおけるオフィス環境下でのストレス状態推定，電子情報通信学会技術研究報告，13 July 2016, vol. 116, no. 141, pp. 7-12, particularly, p. 7, left column to p. 11, left column, (KUBOI, Ryo, Estimating stress states using respiratory monitoring in office environment by wideband radar, IEICE Technical Report) | 11-12 |
| Y | JP 2019-122652 A (JOYSON SAFETY SYSTEMS CO., LTD.) 25 July 2019 (2019-07-25), paragraphs [0012]-[0088] | 12 |
| A | JP 2-49639 A (MATSUSHITA ELECTRIC WORKS, LTD.) 20 February 1990 (1990-02-20), page 1, lower left column, line 20 to lower right column, line 7 | 1-16 |
| A | 江口 佳那 他，日常環境を対象とした推定呼吸特徴量による自律神経活動の状態推定，電子情報通信学会技術研究報告，20 January 2017, vol. 116, no. 435, pp. 57-62, entire text, all drawings, (EGUCHI, Kana et al., Autonomic nervous activity estimation for daily life using estimated respiratory feature derived from heart rate variability features, IEICE Technical Report) | 1-16 |
| A | 太田 楓 他，呼吸変動解析によるストレス指標の提案，情報処理学会研究報告 バイオ情報学 (BIO), 23 June 2015, vol. 2015-BIO-42, no. 22, pp. 1-6, entire text, all drawings, (OTA, Kaede et al., A stress indexing method by respiratory variability analysis), non-official translation (IEICE Technical Report (BIO)) | 1-16 |

Form PCT/ISA/210 (second sheet) (January 2015)

<table>
<tr><td colspan="2" align="center"><strong>INTERNATIONAL SEARCH REPORT</strong><br>Information on patent family members</td><td>International application No.<br>PCT/JP2021/008238</td></tr>
</table>

| | | |
|---|---|---|
| JP 2011-15887 A | 27 January 2011 | FR 2947712 A1 |
| JP 2011-94881 A | 12 May 2011 | FR 2952169 A1 |
| | | FR 2952168 A1 |
| JP 2005-52413 A | 03 March 2005 | (Family: none) |
| JP 2019-122652 A | 25 July 2019 | WO 2019/142720 A1 |
| JP 2-49639 A | 20 February 1990 | (Family: none) |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H08280637 A